(19) Europäisches Patentamt
European Patent Office
Office européen des brevets

(11) Numéro de publication: **0 149 582**
**B1**

# (12) FASCICULE DE BREVET EUROPÉEN

(45) Date de publication du fascicule du brevet: **18.05.88**

(51) Int. Cl.⁴: **C 07 K 5/06**, C 07 C 101/22

(21) Numéro de dépôt: **85400084.1**

(22) Date de dépôt: **18.01.85**

## (54) Procédé de préparation d'aspartyl peptides et nouveaux dérivés de l'acide aspartique utilisables dans leur synthèse.

(30) Priorité: **19.01.84 FR 8400794**
**05.10.84 FR 8415280**

(43) Date de publication de la demande:
**24.07.85 Bulletin 85/30**

(45) Mention de la délivrance du brevet:
**18.05.88 Bulletin 88/20**

(84) Etats contractants désignés:
**AT BE CH DE FR GB IT LI LU NL SE**

(56) Documents cité:
**EP-A-0 035 047**
**EP-A-0 048 345**
**EP-A-0 099 960**
**DE-A-1 143 516**
**US-A-4 332 718**

**TETRAHEDRON LETTERS, no. 7, 1979, pages 613-616, Pergamon Press, GB; J. TSUJI et al.: "A convenient method for the preparation of 1-olefins by the palladium catalyzed hydrogenolysis of allylic acetates and allylic phenyl ethers with ammonium formate"**

(73) Titulaire: **ISOCHEM (société anonyme), 10, rue Clément Marot, F-75008 Paris (FR)**

(72) Inventeur: **Wirth, Didier, 3, place du Palais Bourbon, F-75007 Paris (FR)**
Inventeur: **Gibert, Dominique, 12, rue de l'Eglise, F-60340 Villers- S/Saint- Leu (FR)**
Inventeur: **Boutin, Annie, 3, place André Malraux, F-92390 Villeneuve- La- Garenne (FR)**

(74) Mandataire: **L'Helgoualch, Jean, OFFICE PICARD 134 Boulevard de Clichy, F-75018 Paris (FR)**

**EP 0 149 582 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne un procédé de préparation de divers peptides, et plus particulièrement un nouveau procédé utilisable dans la synthèse industrielle d'aspartyl peptides tels que des édulcorants ou des hormones polypeptidiques, ainsi que les produits nouveaux utilisés comme intermédiaires de synthèse.

On sait que la préparation des aspartyl peptides est particulièrement compliquée du fait des deux fonctions acides non identiques de l'acide aspartique; ainsi, l'anhydride interne benzyloxycarbonyl-aspartique, matière première habituelle (voir W.J. LE QUESNE, J. Chem. Soc. 1952 p.24, ou brevet FR 2.040.473) de la synthèse de ces peptides, conduit à un mélange d' α et β-aspartyl peptides, souvent difficilement séparables, ce qui constitue une perte en acides aminés sous la forme de l'isomère non désiré.

Pour éviter la formation d'un isomère parasite, on utilise parfois un dérivé de l'acide aspartique protégé à la fois sur l'azote et sur l'une des fonctions acides, par exemple le benzyloxycarbonyl-β-aspartate de benzyle (voir Bryant, J. Chem. Soc. 1959 p.3868 ou brevet DE 2.608.174); l'utilisation industrielle d'un tel réactif est cependant limitée par la longueur de sa préparation, le coût des matières premières (hydroxyde de lithium et chloroformiate de benzyle) et les problèmes posés par la déprotection totale du peptide obtenu, l'hydrogénolyse libérant deux molécules de toluène qui bloquent l'opération menée par ailleurs avec avantage en milieu aqueux, par enrobage du catalyseur.

Les documents EP-A-0.035.047 et US-A-4.332.718 décrivent un procédé de préparation d'esters d'aspartyl phényl alanine par réaction d'un composé dicarbonylé sur un sel d'acide aspartique, procurant un mélange des isomères α et β.

Le document DE-A-1.143.516 décrit la préparation de peptides par condensation d'un composé dicarbonylé avec un acide aminé, puis couplage avec un amino-acide par la méthode aux esters de cyanométhyle. Le document EP-A-0.048.345 décrit un procédé de préparation d'esters alkyliques d'aspartyl phényl alanine par protection des fonctions acides et amine de l'acide aspartique, réaction avec un halogénoester, et condensation avec un ester de phényl alanine.

L'utilisation d'esters allyliques comme groupes protecteurs d'acides carboxyliques est décrite dans Tetrahedron Letters n° 7 p.613-616 (1979).

L'invention a pour objet un nouveau procédé de préparation d'aspartyl peptides, utilisable industriellement, procurant un rendement satisfaisant en évitant les inconvénients inhérents aux méthodes connues rappelés ci-dessus.

L'invention a également pour objet un nouveau procédé de préparation d'aspartyl peptides utilisant de nouveaux dérivés de l'acide aspartique efficacement protégés au niveau de l'une des fonctions acides, permettant une déprotection finale sélective réalisable dans des conditions satisfaisantes de sécurité et de rentabilité.

L'invention a également pour objet de nouveaux dérivés de l'acide aspartique utilisables comme intermédiaires dans la synthèse industrielle d'aspartyl peptides.

Conformément au procédé de l'invention, on fait réagir un β-monoester d'acide aspartique de formule générale (I):

$R_1O$-CO-$CH_2$-CH-COOH (I)
|
$NH_2$

dans laquelle $R_1$ représente un radical hydrocarboné portant éventuellement un substituant activateur,

avec un composé β-dicarbonylé de formule (II):

$R_2$-CO-CH-CO-X (II)
|
$R_3$

dans laquelle $R_2$ représente un radical hydrocarboné ou carbométhoxyméthyle ou carboéthoxyméthyle; $R_3$ est un atome d'hydrogène ou forme avec $R_2$ une chaîne $-(CH_2)_n-$ où n vaut 3 ou 4; X représente un radical hydrocarboné ou forme avec $R_2$ (et dans ce cas $R_3$ est un atome d'hydrogène) une chaîne à trois atomes de carbone éventuellement substituée, ou est un radical alcoxy $-OR_4$, $R_4$ étant un radical hydrocarboné, ou encore est un radical $-NR_5R_6$ dans lequel $R_5$ et $R_6$, identiques ou différents, représentent un atome d'hydrogène, un radical hydrocarboné éventuellement substitué, ou forment ensemble un cycle à 5 ou 6 chaînons dont l'un peut être un atome d'oxygène;

pour former une ènamine de formule (III):

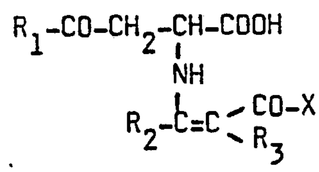

(III)

dans laquelle $R_1$, $R_2$, $R_3$ et X ont la définition indiquée ci-dessus; puis on effectue un couplage avec un amino-acide, un amino ester, un amino amide, ou un peptide, préalablement protégé le cas échéant, et on élimine si nécessaire les groupements protecteurs, pour obtenir l'aspartyl peptide de formule (IV):

HOOC-$CH_2$-CH-CO-NH-R (IV)
|
$NH_2$

où -NHR représente un reste d'amino-acide ou de peptide.

L'expression "radical hydrocarboné" utilisée ci-dessus désigne un radical alcoyle de 1 à 5 atomes

de carbone, linéaire ou ramifié, saturé ou insaturé, ou un radical phényle éventuellement substitué par un radical méthyle, un atome d'halogène ou un radical méthoxy, ou encore un radical benzyle (ou phényl-1 éthyle) éventuellement substitué par un groupe activateur tel qu'un radical méthyle, un radical méthoxy ou un groupe nitro.

Comme indiqué ci-dessus, l'invention concerne également les dérivés d'acide aspartique de formule (III), ainsi que leurs sels.

L'invention concerne notamment les dérivés d'acide aspartique représentés par la formule (III) dans laquelle $R_1$ représente un radical allyle de formule (V):

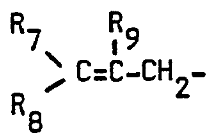

(V)

dans laquelle $R_7$, $R_8$ et $R_9$, identiques ou différents, représentent un atome d'hydrogène, un atome d'halogène, un radical alcoyle ramifié ou non comportant de 1 à 6 atomes de carbone, ou encore un radical phényle.

Les dérivés de formule générale (III) où $R_1$ est un radical allyle de formule (V) peuvent être préparés de plusieurs façons simples et usuelles, par exemple par estérification, à l'aide d'un alcool de formule (VI):

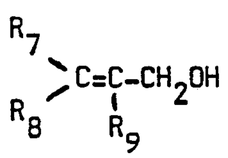

(VI)

d'un acide de formule (VII):

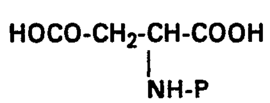

(VII)

dans laquelle P désigne le radical dérivé du composé β-dicarbonylé de formule (II),

ou par O-alcoylation d'un acide de formule (VII) par un agent de formule (VIII):

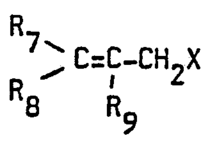

(VIII)

dans laquelle X représente un atome d'halogène ou un groupe alkyl-sulfonyle ou aryl-sulfonyle.

Les réactifs mis en oeuvre sont souvent des produits industriels courants, par exemple les alcools allylique, méthallylique, crotylique, cinnamique et l'éthyl-2 hexène-2-ol-1, les chlorures ou bromures d'allyle, méthallyle, prényle ou cinnamyle, le dichloro-1,3-propène, le dichloro-2,3-propène ou le dichloro-1,3-butène-2.

Les dérivés d'acide aspartique de formule (III) selon l'invention permettent d'obtenir facilement des α-aspartyl peptides β-bloqués par condensation avec des amino acides, des amino esters, des amino amides, ou des peptides, comme indiqué plus en détail ci-après.

Suivant une forme préférentielle de réalisation du procédé de l'invention, on fait réagir le monoester aspartique de formule (I) avec le composé dicarbonylé de formule (II) en présence d'une amine secondaire ou tertiaire pour former l'ènamine de formule (III) que l'on peut alors isoler aisément sous forme de sel d'amine. Cette possibilité d'isoler l'amine peut faciliter son application à diverses synthèses d'aspartyl peptides. Bien entendu, la synthèse peut s'effectuer sans isoler l'ènamine.

La N-protection des monoacides aminés sous forme d'ènamines de composés β-dicarbonylés est bien connue mais on sait que l'application de cette méthode à des monoesters de diacides est délicate, notamment dans le cas du β-glutamate de benzyle (voir A. Balog, Rev. Roum. Chim. 15 (1970) p.1375). Par ailleurs, comme indiqué par R. Hirschmann et al., JACS 93 p.2746 (1971), la protection de l'acide aspartique sous forme de β-aspartate de méthyle ou de benzyle conduit à la formation de β- aussi bien que d'α-aspartyl peptides, sans doute en raison de la formation d'un intermédiaire cyclique, et l'interconversion des deux isomères α et β ne peut être évitée, ce qui constitue un sérieux inconvénient. Ainsi, connaissant la bien plus grande fragilité des dérivés aspartiques comparés aux dérivés glutamiques, il n'était pas prévisible que les ènamines de formule (III) ci-dessus puissent être préparées et mises en oeuvre dans des conditions satisfaisantes pour la synthèse industrielle d'aspartyl peptides.

L'isomèrie optique de l'acide aspartique permet d'obtenir sous les formes D ou L les ènamines de formule (III), et l'invention concerne bien entendu ces deux formes et leurs mélanges. On comprendra aisément que les sels obtenus à l'aide d'une amine optiquement active puissent exister sous deux formes diastéréoisomères, et que ce fait peut être mis à profit pour obtenir des produits de configuration déterminée à partir, par exemple, de dérivés aspartiques de pureté optique plus ou moins grande, ou même racémiques.

Les monoesters aspartiques utilisables comme produits de départ, représentés par la formule (I), sont des corps bien souvent connus tels, par exemple, les β-aspartates de méthyle, tertiobutyle ou benzyle; selon l'utilisation et notamment le mode de déprotection de l'aspartyl peptide préparé, on préfèrera tel ou tel ester; par exemple, pour la synthèse du L-α-aspartyl L-phénylalaninate de méthyle, le β-L-aspartate de benzyle est préféré au β-L-aspartate de méthyle, ce dernier conduisant à un diester dont la saponification sélective s'avère difficile. Il est également avantageux d'utiliser le β-L-aspartate

d'allyle ou de crotyle.

De même, peuvent servir d'agents bloquants de formule (II), des composés β-dicarbonylés divers, souvent usuels, tels par exemple les β-dicétones comme l'acétylacétone, la benzoylacétone, le dibenzoylméthane, la cyclohexanedione-1,3, ou la dimédone, des β-cétoesters comme les acétyl acétates de méthyle, éthyle, butyle, allyle, benzyle ou α-méthylbenzyle, les cyclopentanone et cyclohexanone-2 carboxylates de méthyle ou d'éthyle, le pivaloyl acétate de méthyle ou l'acétone dicarboxylate de méthyle ou d'éthyle, des β-cétoamides comme le N,N-diméthylacétylacétamide, l'acétylacétanilide ou l'o-méthoxyacétylacétanilide; pour des raisons de commodité industrielle, on utilise de préférence les β-cétoesters et plus particulièrement l'acétyl acétate d'éthyle.

On peut employer comme amines des amines secondaires de préférence, bien que ce ne soit pas un nécessité absolue, encombrées au niveau de l'atome d'azote, comme la diisopropylamine, la N-méthylcyclohexylamine, la dicyclohexylamine, la N-tétraméthyl-2,2,6,6 pipéridine et la N-méthyl D ou L-α-méthylbenzylamine, ou des amines tertiaires comme la triéthylamine, la tributylamine, la N-méthylmorpholine, la N-méthylpipéridine, et la N,N-diméthyl D ou L-α-méthylbenzylamine, par exemple. La dicyclohexylamine est souvent préférée lorsque l'on désire isoler les ènamines de formule (III) sous forme pure cristallisée.

La réaction du monoester d'acide aspartique de formule (I) avec le composé β-dicarbonylé de formule (II), le cas échéant en présence d'amine, s'effectue de préférence dans un solvant qui peut être un alcool, tel que le méthanol, l'éthanol, l'isopropanol ou un butanol, par exemple, un éther comme l'éther diéthylique, l'éther diisopropylique, l'éther de méthyle et de tertiobutyle, le tétrahydrofuranne ou le dioxanne, un ester comme les acétates d'éthyle, d'isopropyle ou de butoxy-2 éthyle, un hydrocarbure aromatique ou non comme le toluène, le xylène, l'heptane ou le cyclohexane, une cétone comme l'acétone, la méthyléthylcétone, un dérivé halogéné comme le chlorure de méthylène, le chloroforme, le dichloro-1,2 éthane ou le chlorobenzène, l'acétonitrile ou un solvant polaire aprotique tel que le diméthylformamide, le diméthylacétamide, la N-méthylpyrrolidone, le diméthylsulfoxyde, l'hexaméthylphosphotriamide ou encore un mélange de ces solvants. On utilise de préférence un solvant dans lequel les réactifs, solubles au départ, conduisent à un sel insoluble si l'on désire isoler celui-ci.

La réaction, souvent spontanée à température ambiante, peut être menée à température plus élevée, et jusqu'à 130°C environ dans le but de diminuer les durées opératoires, d'augmenter la solubilité des réactifs ou encore d'éliminer l'eau formée par distillation azéotropique avec le solvant. Cette élimination de l'eau peut être obtenue aussi à l'aide d'adjuvants compatibles avec les conditions de réaction, par exemple des tamis moléculaires 3Å.

Les réactifs peuvent être opposés en quantités stoechiométriques, mais on préfère utiliser un excès d'amine et de composé β-dicarbonylé par rapport à l'acide aspartique. Les ènamines obtenues sont isolables par essorage, éventuellement après concentration du milieu réactionnel, et désolubilisation à l'aide d'un second solvant; elles peuvent aussi être utilisées brutes en solution pour effectuer directement le couplage avec l'amino-acide ou le peptide.

Les ènamines de formule (II) sont le plus souvent des corps solides, stables et caractérisés par leur point de fusion, leurs spectres d'absorption infrarouge et ultraviolette et de résonance magnétique nucléaire, de même que par leurs constantes chromatographiques (chromatographie sur couche mince ou chromatographie liquide haute performance).

La réaction de couplage de l'ènamine de formule (III) avec un amino-acide, un ester ou un peptide s'effectue par les méthodes usuelles en synthèse peptidique, par exemple par la méthode au dicyclohexylcarbodiimide, la méthode aux esters de cyanométhyle ou, de préférence, la méthode aux anhydrides mixtes.

Dans le premier cas, on ajoute le dicyclohexylcarbodiimide à un mélange de l'ènamine de formule (III) et d'un sel, par exemple le chlorhydrate, de l'aminoester à acyler; l'aspartyl peptide N-protégé est obtenu après élimination de la dicyclohexylurée par filtration.

Dans le cas de la méthode aux esters de cyanométhyle, il convient de préparer au préalable l'ester activé du β-aspartate N-protégé; ceci est réalisé par contact de l'ènamine de formule (III) avec du chloroacétonitrile, ou mieux du tosyloxyacétonitrile dans un solvant aprotique; après isolement de l'ester de cyanométhyle, on oppose celui-ci à l'aminoester à acyler dans les conditions classiques (voir G. Wendlberger in Houben-Weyl "Methoden der organischen Chemie" 1974 15/2 p.8).

La méthode aux anhydrides mixtes semble d'application particulièrement simple: l'ènamine de formule (III) est traitée par un chlorure d'acide ou un chloroformiate dans un solvant approprié et l'anhydride mixte intermédiairement obtenu traité par l'aminoester à acyler sous forme de base libre ou de sel, par exemple le chlorhydrate, dont on le libère par addition supplémentaire d'un équivalent de base. On obtient ainsi directement en solution le peptide N-protégé. Les chlorures d'acide utilisables sont par exemple le chlorure d'acétyle, le chlorure de pivaloyle ou le chlorure de benzoyle; les chloroformiates de méthyle, éthyle, isopropyle ou isobutyle conviennent aussi; on préfère le chloroformiate d'éthyle.

Quelle que soit la méthode de couplage utilisée, on passe ensuite au peptide N-déprotégé par une simple coupure de l'ènamine à

l'aide d'un acide, par exemple l'acide chlorhydrique normal. Les α-aspartyl peptides ainsi obtenus sont très purs et exempts de l'isomère β, ce qui signifie que l'interconversion des deux isomères, souvent constatée dans les synthèses classiques, n'a pas lieu ici. Ce résultat inattendu est particulièrement important et rend le procédé de l'invention très avantageux par rapport aux procédés usuels.

La condensation de l'ènamine de formule (III) avec des amino acides, des amino esters, des amino amides ou des peptides pour former des aspartyl peptides, peut s'effectuer en une seule étape par les méthodes de couplage connues mentionnées ci-dessus. De plus, notamment lorsque le groupe $R_1$ dans la formule (III) est un radical allyle de formule (V), on peut également procéder en deux étapes, dont la première consiste en la préparation d'un nouvel intermédiaire activé isolable comme un α-ester de formule générale (IX):

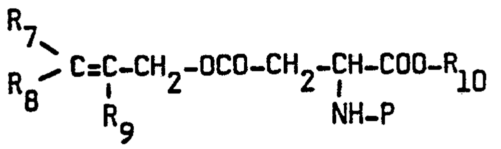
(IX)

dans laquelle $R_{10}$ désigne un radical tel que cyanométhyle, nitro-2 (ou-4) phényle, trichloro-2,4,6 (ou-2,4,5) phényle, phénylazo-4-phényle ou N-succinimido, et P désigne le radical dérivé du composé β-dicarbonylé de formule (II).

On obtient ainsi facilement les α-aspartyl peptides de formule générale (X):

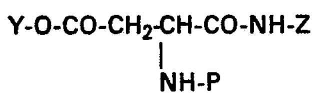
(X)

dans laquelle Y représente le reste de formule (V) ci-dessus, P a la même signification que dans la formule (VI), et Z représente un reste d'acide aminé, d'amino ester, d'amino amide ou de peptide.

Enfin, les α-aspartyl peptides β-bloqués de formule indiquée ci-dessus, obtenus selon l'invention sont aisément convertis en α-aspartyl peptides β-libres représentés par la même formule ou Y est un atome d'hydrogène par simple protolyse en présence d'un catalyseur à base de métal de transition; cette possibilité est l'un des avantages majeurs procurés par les dérivés de formule (III) et leur mise en oeuvre lors de synthèses peptidiques.

Cette β-déprotection peut être effectuée par exemple en milieu protique, aqueux ou non, et entre -30° C et +100° C environ mais de préférence à température ambiante; dans certains cas l'utilisation d'un solvant non miscible à l'eau peut se révéler favorable. La catalyse peut être hétérogène ou homogène: conviennent ainsi les catalyseurs formés par les métaux de transition en poudre pure ou supportés (par du carbone, de l'alumine ou de la silice par exemple) comme le palladium ou le ruthénium utilisés classiquement pour les hydrogénations, mais aussi des sels ou des complexes solubles de ces métaux comme le complexe de Wilkinson ou les catalyseurs de réduction asymétrique homogène.

Cette possibilité de séparer aisément le groupe protecteur allylique de la fonction acide en β du reste aspartique est avantageuse et inattendue en raison de l'existence des autres groupes protecteurs qui ne sont pas modifiés.

Cette nouvelle synthèse d'α-aspartyl peptides β-libres à partir des dérivés de formule (III) utilisés comme intermédiaires de synthèse, conformément à la présente invention, est particulièrement avantageuse car sa mise en pratique apparaîtra simple à l'homme de l'art.

Les nouvelles ènamines de formule (III) conformes à la présente invention constituent donc des intermédiaires particulièrement utiles et avantageux en synthèse peptidique industrielle, en raison notamment de leur grande facilité de préparation à partir de produits de départ facilement accessibles et de leur réactivité suffisante, contrairement aux produits connus utilisés dans les méthodes classiques.

Plus particulièrement, il est possible de préparer dans d'excellentes conditions des peptides tels que des angiotensines diverses utiles en thérapeutique humaine et vétérinaire, l'aspartame, la pentagastrine, la phyllomedusine, l'upéroléine, l'eledoïsine ou la physalaemine. Ces peptides connus peuvent être préparés à partir des ènamines de formule (III) de l'invention, en plusieurs étapes, suivant les méthodes usuelles en synthèse peptidique. Par exemple le N-L-α-aspartyl L-phénylalaninamide et la N-L-α-aspartyl L-alanine, obtenus directement à partir d'une ènamine de formule (III), sont des intermédiaires de synthèse de la pentagastrine et de l'eledoïsine respectivement.

Les exemples suivants illustrent l'invention plus en détail sans en limiter la portée.

**Exemple 1**

β-L-aspartate d'allyle

On chauffe pendant 6 heures à 60° C un mélange de 133 g d'acide L-aspartique, 56 ml d'acide sulfurique à 96 % et 400 ml d'alcool allylique puis on ajoute lentement environ 500 ml de soude aqueuse à 12 % et on concentre sous vide jusqu'à obtention d'une pâte épaisse. On ajoute alors 1 litre de butanol et de l'eau jusqu'à solubilisation totale; on décante et extrait la phase aqueuse avec 500 ml de butanol.

Les phases butanoliques réunies sont concentrées sous vide; on essore et rince avec 300 ml de butanol froid les cristaux: on obtient après séchage 61 g de produit brut soit un rendement de 35 %.

Un traitement par l'éthanol aqueux permet d'obtenir le β-L-aspartate d'allyle pur.

F = 192° C
Rf = 0,45 (méthyléthyl cétone: 45; eau: 15; acide acétique: 15)
L'acide L-aspartique et l'alcool allylique n'ayant pas réagi sont récupérés et recyclés.
Dans les mêmes conditions que ci-dessus, l'alcool crotylique (butène-2-ol-1) conduit avec un rendement de 25 % au β-L-aspartate de crotyle pur.
Rf = 0,56 (méthyléthyl cétone: 45; eau: 15; acide acétique: 15)

**Exemple 2**

Sel de dicyclohexylamine du N-(carbéthoxy-2 méthyl-1 vinyl)
β-L-aspartate de benzyle
On charge dans un réacteur de 4 litres, 223 g de β-L-aspartate de benzyle, 260 g d'acétyl acétate d'éthyle, 271 g de dicyclohexylamine et 3 litres de toluène sec. Après 48 heures d'agitation à température ambiante, on concentre sous vide au maximum, puis on ajoute 3 litres d'heptane et on agite pendant 6 heures à +10° C environ. On essore et on rince avec 2 litres d'heptane. Après séchage, on obtient 455 g de produit blanc, soit un rendement de 88 %.
F = 97° C
Masse moléculaire équivalente (dosage $HC10_4$) = 514 (théorie = 516)

**Exemple 3**

Sel de dicyclohexylamine du N-carbéthoxy-2 méthyl-1 vinyl)
β-L-aspartate de tertiobutyle
On charge dans un réacteur de 500 ml 18,9 g de β-L-aspartate de tertiobutyle, 26 g d'acétyl acétate d'éthyle, et 20 g de dicyclohexylamine, puis 200 ml d'heptane. Après 24 heures d'agitation, on filtre, on rince avec 100 ml d'heptane et on sèche. On obtient ainsi 7,7 g de produit blanc, soit un rendement de 16 %; un second jet peut s'obtenir par concentration du filtrat.
F = 124°° C.

**Exemple 4**

Sel de diisopropylamine du N-(carbéthoxy-2 méthyl-1 vinyl)
β-L-aspartate de benzyle
On charge dans un réacteur de 500 ml 22,3 g de β-L-aspartate de benzyle, 26 g d'acétyl acétate d'éthyle, et 11,3 g de diisopropylamine anhydre. Après 72 heures d'agitation à température ambiante, on décante l'huile obtenue qui cristallise après quelques heures d'agitation en présence de 300 ml d'heptane. On filtre, on rince et on sèche pour obtenir 34 g de produit blanc (rendement 77,6 %).
F = 100° C.

**Exemple 5**

N-(carbéthoxy-2 méthyl-1 vinyl) L-aspartate de benzyle et d'α-cyanométhyle
On charge dans un réacteur de 500 ml 51,6 g du composé obtenu selon l'exemple 2, 216 g de tosyloxyacétonitrile, 250 ml d'acétone et 100 ml de diméthylformamide. Après 24 heures d'agitation à température ambiante, on ajoute 100 ml d'eau et on refroidit à 0° C environ, puis on filtre et on rince à l'eau. Après séchage, on obtient le produit attendu avec un rendement de 65 %.
F = 104° C.

**Exemple 6**

N-(carbométhoxy-2 méthyl-1 vinyl) β-L-aspartate d'allyle
On agite pendant 4 jours à température ambiante un mélange de 34,6 g de β-L-aspartate d'allyle, 35g d'acétylacétate de méthyle, 25 g de diisopropylamine et 175 ml d'heptane. On filtre, rince à l'heptane et sèche pour obtenir 53 g du produit attendu sous forme de sel de diisopropylamine, soit un rendement de 71 %.
F = 83° C

**Exemple 7**

L-(β-allyl aspartyl) L-phénylalaninate de méthyle
A une suspension de 29 g de chlorhydrate de L-phénylalaninate de méthyle et 50 g du composé précédemment obtenu dans 250 ml de chloroforme on ajoute successivement vers -20° C 25 ml d'oxychlorure de phosphore et 36 ml de triéthylamine; après deux heures d'agitation entre -20° C et 0° C, on hydrolyse avec 200 ml d'eau, décante puis concentre sous vide la phase chloroformique. L'huile résiduelle est traitée par l'acide oxalique dans l'isopropanol, on obtient ainsi 27,9 g de L-(β-allyl aspartyl) L-phénylalaninate de méthyle sous forme d'oxalate (1 : 1).
F = 152° C
Rf = 0,6 (Toluène: 90; méthanol: 10; diéthylamine: 10)

**Exemple 8**

L-α-aspartyl L-phénylalaninate de méthyle
On procède comme dans l'exemple précédent

et le résidu huileux obtenu est mis en solution dans 500 ml de méthanol à 10 % d'eau puis agité pendant 12 heures vers 30° C en présence de 3 g de charbon palladié à 10 %. Un contrôle chromatographique montre que le β-allyl peptide a disparu; après élimination du catalyseur, on ajuste le pH à 5,3 et isole après sèchage 27 g de L-α-aspartyl L-phénylalaninate de méthyle soit un rendement de 70 %. Le produit est identique à un échantillon d'aspartame commercial.

**Exemple 9**

L-α-aspartyl L-phénylalaninate de méthyle

Dans un réacteur de 1 litre on charge 51,6 g du composé obtenu selon l'exemple 2, 500 ml d'acétate d'isopropyle et 70 ml de N,N-diméthylacétamide, puis on refroidit à -20° C environ.

On coule en 1 heure 10,1 ml de chloroformiate d'éthyle, puis on agite encore pendant 1 heure à -20° C.

On charge 21,5 g de chlorhydrate de L-phénylalaninate de méthyle, puis on coule en 1 heure 10,1 g de N-méthylmorpholine. On laisse ensuite remonter la température vers +10° C en 3 heures.

On charge 300 ml d'acide chlorhydrique normal, puis on agite pendant 12 heures à 20° C environ. On décante et on lave deux fois la phase organique à l'acide chlorhydrique normal, puis on réunit les phases aqueuses. A ce stade, la phase aqueuse contient du L-β-benzyl α-aspartyl L-phényl alaninate de méthyle, identifiable par son oxalate (F = 170° C).

On ajoute 6 g de noir de carbone palladié à 5 % et on hydrogène sous 2 bars pendant 2 heures environ. Après élimination du catalyseur et élévation du pH jusqu'à 5,3, on filtre, on rince et on sèche pour obtenir 14 g du produit recherché (rendement 32 %).

$[\alpha]^{20}_D$ = 13,1° CLHP : 1 seul pic.

**Exemple 10**

L-α-aspartyl L-phénylalaninate de méthyle (2 éme méthode)

Dans un réacteur de 500 ml, on charge 35 g du composé obtenu selon l'exemple 4, 17,25 g de chlorhydrate du L-phénylalaninate de méthyle et 170 ml d'acétyl acétate d'éthyle. A la suspension ainsi obtenue et refroidie à 0° C, on ajoute lentement 16,5 g de dicyclohexylcarbodiimide puis on agite encore pendant 2 heures à 0° C et 1 heure vers 20° C.

On ajoute 80 ml d'eau, puis on amène le pH de 4 à 1 à l'aide d'acide chlorhydrique; après 12 heures d'agitation à température ambiante, on élimine la dicyclohexylurée par filtration on décante et extrait phase organique à l'aide de 50 ml d'acide chlorhydrique N; aux phases aqueuses réunies, on ajoute lg de palladium à 5 % sur charbon, et on agite pendant 3 heures sous atmosphère d'hydrogène.

Un dosage chromatographique montre que le milieu contient alors 23 g du produit recherché (rendement 35 %).

**Exemple 11**

L-α-aspartyl L-phényl alaninate de méthyle (3 ème méthode)

Dans un réacteur de 1 litre, on charge 500 ml d'acétate d'isopropyle et 51,6 g du composé obtenu selon l'exemple 2, puis on refroidit à -20° C. On coule en 20 mn 14,5 g de chlorure de pivaloyle et on maintient encore pendant 30 mn à -20° C.

On charge 21,6 g de chlorhydrate de L-phényl alaninate de méthyle puis on coule en 30 mn 10,1 g de N-méthyl morpholine. On agite encore pendant 1 heure à -20° C, puis 1 heure à +20° C.

L'hydrolyse et l'hydrogénolyse suivant les techniques usuelles permettent d'isoler 4 g de L-α-aspartyl L-phényl alaninate de méthyle.

CLHP: 1 seul pic.

**Exemple 12**

N-L-α-aspartyl L-alanine

On charge dans un réacteur de 500 ml 25,8 g du composé obtenu selon l'exemple 2, et 250 ml d'acétate d'isopropyle, puis on refroidit vers -20° C. On coule en 25 mn 5 ml de chloroformiate d'éthyle, puis on agite encore pendant 30 mn vers -20° C.

On charge alors rapidement 16,6 g de tosylate du L-alaninate de benzyle, puis on coule en 15 mn vers -15° C, 5,1 g de N-méthyl morpholine. Après 3 heures supplémentaires d'agitation entre -15 et +20° C, on ajoute 100 ml d'acide chlorhydrique normal et on abandonne pendant 24 heures à température ambiante.

Après filtration et réduction catalytique dans les conditions habituelles on obtient 3,2 g de L-α-aspartyl L-alanine.

F = 194 - 196° C.

**Exemple 13**

N-α-aspartyl L-phénylalaninamide

On charge dans un réacteur de 500 ml 25,8 g du composé obtenu selon l'exemple 2, et 250 ml d'acétate d'éthyle, puis on refroidit vers -25° C.

On coule en 30 mn 5 ml de chloroformiate d'éthyle, puis on maintient encore pendant 30 mn vers -15° C. On ajoute 10 g du chlorhydrate du phénylalaninamide, puis on coule en 20 mn vers -15° C 5,1 g de N-méthyl morpholine.

Le traitement habituel permet d'obtenir 6 g de

N-α-aspartyl L-phénylalaninamide.
F = 190°C.

**Exemple 14**

N-benzyloxycarbonyl L-β-aspartate d'allyle

A un mélange vigoureusement agité de 800 ml d'eau, 138 g de carbonate de potassium et 86,5 g de L-β-aspartate d'allyle, on ajoute lentement 80 ml de chloroformiate de benzyle. Après 2 heures on extrait le produit recherché avec 500 ml de chloroforme du milieu réactionnel acidifié au préalable à pH = 1 à l'aide d'HCl concentré. On élimine le chloroforme par distillation à sec puis dissout le résidu dans 1 litre d'isopropanol et ajoute 90 g de dicyclohexylamine. On filtre, rince à l'isopropanol et sèche pour finalement obtenir 105 g de sel de dicyclohexylamine du N-benzyloxycarbonyl L-β-aspartate d'allyle.

F = 114°C

Par condensation avec le L-phényl-alaninate de méthyle ce corps conduit après addition de ruthénium à 5 % sur carbone, puis hydrogénation, à l'aspartame avec un rendement satisfaisant.

**Revendications**

1. Procédé de préparation d'α-aspartyl peptides de formule générale (IV) exempts d'isomère β:

HOOC-CH$_2$-CH -CO-NH-R (IV)
|
NH$_2$

dans laquelle -NHR représente un reste d'amino-acide ou de peptide, caractérisé en ce que l'on fait réagir un β-monoester d'acide aspartique de formule générale (I):

R$_1$O-CO-CH$_2$-CH-COOH (I)
|
NH$_2$

dans laquelle R$_1$ représente un radical hydrocarboné portant éventuellement un substituant activateur,

avec un composé β-dicarbonylé de formule (II):

R$_2$-CO-CH-CO-X (II)
|
R$_3$

dans laquelle R$_2$ représente un radical hydrocarboné ou carbométhoxyméthyle ou carboéthoxyméthyle; R$_3$ est un atome d'hydrogène ou forme avec R$_2$ une chaîne $-(CH_2)_n-$ où n vaut 3 ou 4; X représente un radical hydrocarboné ou forme avec R$_2$ (et dans ce cas R$_3$ est un atome d'hydrogène) une chaîne à trois atomes de carbone éventuellement substituée, ou est un radical alcoxy -OR$_4$, R$_4$ étant un radical hydrocarboné, ou encore est un radical -NR$_5$R$_6$ dans lequel R$_5$ et R$_6$, identiques ou différents, représentent un atome d'hydrogène, un radical hydrocarboné éventuellement substitué, ou forment ensemble un cycle à 5 ou 6 chaînons dont l'un peut être un atome d'oxygène;

pour former une ènamine de formule (III):

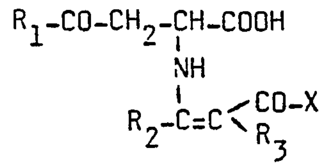
(III)

dans laquelle R$_1$, R$_2$, R$_3$ et X ont la définition indiquée ci-dessus; puis on effectue un couplage avec un amino-acide, un amino ester, un amino amide, ou un peptide, préalablement protégé le cas échéant, et on élimine si nécessaire les groupements protecteurs, la réaction du monoester de formule (I) avec le composé de formule (II) s'effectuant en absence de toute base minérale lorsque R$_1$ est un groupe arylméthyle.

2. Procédé selon la revendication 1, caractérisé en ce que R$_1$ représente un radical allyle de formule (V):

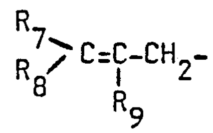
(V)

dans laquelle R$_7$, R$_8$ et R$_9$, identiques ou différents, représentent un atome d'hydrogène, un atome d'halogène, un radical alcoyle ramifié ou non de 1 à 6 atomes de carbone, ou encore un radical phényle.

3. Procédé selon l'une quelconque des revendications 1 et 2, caractérisé en ce que l'on fait réagir le monoester d'acide aspartique de formule (I) avec le composé dicarbonylé de formule (II) en présence d'une amine secondaire ou tertiaire.

4. Procédé selon la revendication 3, caractérisé en ce que l'amine secondaire ou tertiaire est choisie parmi la diisopropylamine, la N-méthylcyclohexylamine, la dicyclohexylamine, la N-tétraméthyl-2,2,6,6 pipéridine, la N-méthyl D- ou L-α-méthylbenzylamine, la triéthylamine, la tributylamine, la N-méthylmorpholine, la N-méthylpipéridine, et la N,N-diméthyl D- ou L-α-méthylbenzylamine.

5. Procédé selon l'une quelconque des revendications 1 à 4, caractérisé en ce que la réaction du monoester d'acide aspartique de formule (I) avec le composé β-dicarbonylé de formule (II) s'effectue dans un solvant choisi parmi un alcool, un éther linéaire ou cyclique, un

ester, un hydrocarbure aromatique ou non, une cétone, un dérivé halogéné ou un solvant polaire aprotique tel que le diméthylformamide, le diméthylacétamide, la N-méthylpyrrolidone, le diméthylsulfoxyde, l'hexaméthylphosphotriamide ou encore un mélange de ces solvants.

6. Procédé selon l'une quelconque des revendications 1 et 2, caractérisé en ce que le monoester aspartique de formule (I) est choisi parmi le β-L-aspartate de benzyle, le β-L-aspartate de méthyle et le β-L-aspartate de tertiobutyle et le β-L-aspartate d'allyle.

7. Procédé selon la revendication 1, caractérisé en ce que le composé dicarbonylé de formule (II) est l'acétylacétate de méthyle, d'éthyle, d'isopropyle ou de butyle.

8. Procédé selon la revendication 2, caractérisé en ce que l'élimination du groupement protecteur en β du reste aspartique est effectuée par protolyse en présence d'un catalyseur à base de métal de transition.

9. Procédé selon la revendication 8, caractérisé en ce que la déprotection est effectuée en milieu protique à une température comprise entre -30° C et +100° C.

10. Procédé selon l'une quelconque des revendications 8 et 9, caractérisé en ce que le métal de transition est le palladium ou le ruthénium.

11. Nouveaux dérivés de l'acide aspartique utiles pour la synthèse d'aspartyl peptides, constitués par les énamines de formule (III):

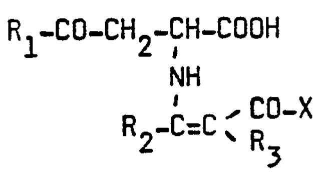 (III)

dans laquelle $R_1$ représente un radical hydrocarboné portant éventuellement un substituant activateur, $R_2$ représente un radical hydrocarboné ou carbométhoxyméthyle ou carboéthoxyméthyle; $R_3$ est un atome d'hydrogène ou forme avec $R_2$ une chaîne $-(CH_2)_n-$ où n vaut 3 ou 4; X représente un radical hydrocarboné ou forme avec $R_2$ (et dans ce cas $R_3$ est un atome d'hydrogène) une chaîne à trois atomes de carbone éventuellement substituée, ou est un radical alcoxy $-OR_4$, $R_4$ étant un radical hydrocarboné, ou encore est un radical $-NR_5R_6$ dans lequel $R_5$ et $R_6$, identiques ou différents représentent un atome d'hydrogène, un radical hydrocarboné éventuellement substitué, ou forment ensemble un cycle à 5 ou 6 chaînons dont l'un peut être un atome d'oxygène; ainsi que leurs sels d'amines.

12. Dérivés de l'acide aspartique selon la revendication 11, caractérisé en ce que $R_1$ représente un radical allyle de formule (V):

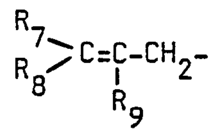 (V)

dans laquelle $R_7$, $R_8$ et $R_9$, identiques ou différents, représentent un atome d'hydrogène, un atome d'halogène, un radical alcoyle ramifié ou non de 1 à 6 atomes de carbone, ou encore un radical phényle.

13. Dérivés de l'acide aspartique selon la revendication 12, caractérisé en ce que $R_7$, $R_8$ et $R_9$ représentent un atome d'hydrogène.

14. Dérivés selon la revendication 11, caractérisés en ce qu'ils sont sous forme de sels d'amine secondaire ou tertiaire.

**Patentansprüche**

1. Verfahren zur Herstellung von α-Aspartylpeptiden der allgemeinen Formel (IV), die frei an β-Isomeren sind:

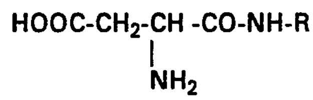 (IV)

worin -NHR einen Aminosäure- oder Peptidrest bedeutet, dadurch gekennzeichnet, daß man einen β-Monoester der Asparaginsäure der allgemeinen Formel (I)

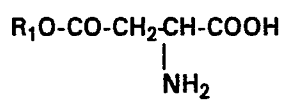 (I)

worin $R_1$ einen Kohlenwasserstoffrest, der gegebenenfalls einen Aktivatorsubstituenten trägt, bedeutet, umsetzt mit einer β-Dicarbonylverbindung der Formel (II)

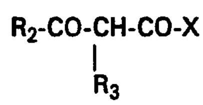 (II)

worin $R_2$ einen Kohlenwasserstoffrest oder Carbomethoxyethyl oder Carboethoxymethyl bedeutet; $R_3$ ein Wasserstoffatom ist oder mit $R_2$ eine Kette $-(CH_2)_n-$, worin n 3 oder 4 sein kann, bildet; X einen Kohlenwasserstoffrest bedeutet oder mit $R_2$ (wobei in diesem Fall $R_3$ ein Wasserstoffatom ist) eine Kette mit 3 gegebenenfalls susbstituierten Kohlenstoffatomen bildet oder einen Alkoxyrest-$OR_4$, wobei $R_4$ einen Kohlenwasserstoffrest darstellt, oder einen Rest $-NR_5R_6$ bedeutet, worin $R_5$ und $R_6$, die gleich oder verschieden sind, ein Wasserstoffatom oder einen gegebenenfalls substituierten Kohlenwasserstoffrest bedeuten oder miteinander einen Ring mit 5 oder 6 Gliedern, von denen eines ein Sauerstoffatom sein kann, bilden;

zur Bildung eines Enamins der Formel (III)

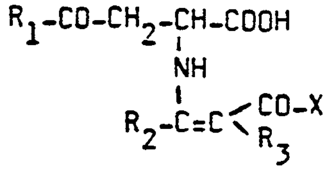

(III)

worin $R_1$, $R_2$, $R_3$ und X die obengenannten Bedeutungen besitzen;

danach eine Kupplung mit einer Aminosäure, einem Aminosäureester, einem Aminosäureamid oder einem Peptid, das erforderlichenfalls vorher geschützt wurde, durchführt und die Schutzgruppen, falls erforderlich, eliminiert, wobei die Umsetzung des Mononesters der Formel (I) mit der Verbindung der Formel (II) in Abwesenheit jeglicher mineralischer Base durchgeführt wird, wenn $R_1$ eine Arylmethylgruppe ist.

2. Verfahren nach Anspruch 1, dadurch gekennzeichnet, daß $R_1$ einen Allylrest der Formel (V) bedeutet:

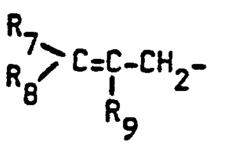

(V)

worin $R_7$, $R_8$ und $R_9$, die gleich oder verschieden sind, ein Wasserstoffatom, Halogenatom, einen gegebenenfalls verzweigten Alkoylrest mit 1 bis 6 Kohlenstoffatomen oder einen Phenylrest bedeuten.

3. Verfahren nach Anspruch 1 oder 2, dadurch gekennzeichnet, daß man den Monoester der Asparaginsäure der Formel (I) mit der Dicarbonylverbindung der Formel (II) in Gegenwart eines sekundären oder tertiären Amins umsetzt.

4. Verfahren nach Anspruch 3, dadurch gekennzeichnet, daß das sekundäre oder tertiäre Amin gewählt wird aus Diisopropylamin, N-Methylcyclohexylamin, Dicyclohexylamin, N-Tetramethyl-2,2,6,6-piperidin, N-Methyl-D- oder L-α-methylbenzylamin, Triethylamin, Tributylamin, N-Methylmorpholin, N-Methylpiperidin und N,N-Dimethyl-D-oder L-α-methylbenzylamin.

5. Verfahren nach einem der Ansprüche 1 bis 4, dadurch gekennzeichnet, daß die Umsetzung des Monoesters der Asparaginsäure der Formel (I) mit der α-Dicarbonylverbindung der Formel (II) in einem Lösungsmittel durchgeführt wird, gewählt aus einem Alkohol, linearen oder zyklischen Ether, Ester, Kohlenwasserstoff, der aromatisch sein kann, Keton, einem halogenierten Derivat oder einem polaren, aprotischen Lösungsmittel, wie Dimethylformamid, Dimethylacetamid, N-Methylpyrrolidon, Dimethylsulfoxid, Hexamethylphosphotriamid oder einer Mischung dieser Lösungsmittel.

6. Verfahren nach Anspruch 1 oder 2, dadurch gekennzeichnet, daß der Asparaginsäuremonoester der Formel (I) gewählt wird aus Benzyl-β-L-aspartat, Methyl-β-L-aspartat, tert-Butyl-β-L-aspartat und Allyl-β-L-aspartat.

7. Verfahren nach Anspruch 1, dadurch gekennzeichnet, daß die Dicarbonylverbindung der Formel (II) Methyl-, Ethyl-, Isopropyl- oder Butylacetylacetat ist.

8. Verfahren nach Anspruch 2, dadurch gekennzeichnet, daß die Eliminierung der Schutzgruppe in β-Stellung des Aspartylrestes mittels Protolyse in Gegenwart eines Katalysators aus einer Base eines Übergangsmetalls durchgeführt wird.

9. Verfahren nach Anspruch 8, dadurch gekennzeichnet, daß die Schutzgruppenelimination in einem protischen Milieu bei einer Temperatur zwischen -30° C und + 100° C durchgeführt wird.

10. Verfahren nach Anspruch 8 oder 9, dadurch gekennzeichnet, daß das Übergangsmetall Palladium oder Ruthenium ist.

11. Neue Derivate der Asparaginsäure, die sich zur Synthese von Aspartylpeptiden eignen, bestehend aus Enaminen der Formel (III)

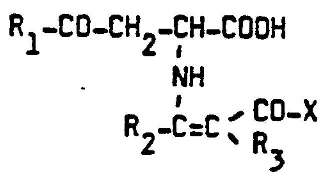

(III)

worin $R_1$ einen Kohlenwasserstoffrest, der gegebenenfalls einen Aktivatorsubstituenten trägt, bedeutet, $R_2$ einen Kohlenwasserstoffrest oder Carbomethoxymethyl oder Carboethoxymethyl bedeutet; $R_3$ ein Wasserstoffatom ist oder mit $R_2$ eine Kette $-(CH_2)_n-$, worin n 3 oder 4 sein kann, bildet; X einen Kohlenwasserstoffrest bedeutet oder mit $R_2$ (wobei in diesem Fall $R_3$ ein Wasserstoffatom ist) eine Kette mit 3 gegebenenfalls substituierten Kohlenstoffatomen bildet oder einen Alkoxyrest $-OR_4$, wobei $R_4$ einen Kohlenwasserstoffrest darstellt, oder einen Rest $-NR_5R_6$ bedeutet, worin $R_5$ und $R_6$, die gleich oder verschieden sind, ein Wasserstoffatom oder einen gegebenenfalls substituierten Kohlenwasserstoffrest bedeuten oder miteinander einen Ring mit 5 oder 6 Gliedern, von denen eines ein Sauerstoffatom sein kann bilden; sowie deren Aminsalze.

12. Derivate der Asparaginsäure nach Anspruch 11, dadurch gekennzeichnet, daß $R_1$ einen Allylrest der Formel (V) bedeutet:

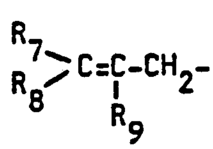

(V)

worin $R_7$, $R_8$ und $R_9$, die gleich oder verschieden sind, ein Wasserstoffatom, Halogenatom, einen gegebenenfalls verzweigten Alkoylrest mit 1 bis 6 Kohlenstoffatomen oder einen Phenylrest bedeuten.

13. Derivate der Asparaginsäure nach Anspruch 12, dadurch gekennzeichnet, daß $R_7$, $R_8$, und $R_9$ ein Wasserstoffatom bedeuten.

14. Derivate nach Anspruch 11, dadurch gekennzeichnet, daß sie in Form von Salzen mit einem sekundären oder tertiären Amin vorliegen.

## Claims

1. Method for the preparation of α-aspartyl peptides of general formula (IV) free of β-isomer:

```
HOOC-CH2-CH-CO-NH-R          (IV)
         |
         NH2
```

wherein -NHR represents an amino-acid or peptide residue, characterized in that it comprises the steps of

reacting a β-monoester of aspartic acid of general formula (I)

```
R1O-CO-CH2-CH-COOH           (I)
           |
           NH2
```

wherein $R_1$ represents a hydrocarbon radical possibly substituted by an activating group,

with a β-dicarbonyl group of general formula (II):

```
R2-CO-CH-CO-X                (II)
        |
        R3
```

wherein $R_2$ represents a hydrocarbon radical, a carbomethoxymethyl radical or a carboethoxyethyl radical; $R_3$ is a hydrogen atom or $R_2$ and $R_3$ are combined to form a $-(CH_2)_n-$ chain wherein n is 3 or 4; X represents a hydrocarbon radical or X and $R_2$ are combined to form a three-carbon chain possibly substituted (and in such case, $R_3$ is a hydrogen atom), or X is a $-OR_4$ alkoxy radical, where $R_4$ is a hydrocarbon radical, or is a $-NR_5R_6$ radical where $R_5$ and $R_6$, which are the same or different, represent a hydrogen atom, a hydrocarbon radical possibly substituted, or are combined to form a 5 or 6-membered ring which may comprise an oxygen atom;

to obtain an enamine of general formula (III):

```
R1-CO-CH2-CH-COOH
          |
          NH
          |
       R2-C=C-CO-X           (III)
            |
            R3
```

where $R_1$, $R_2$, $R_3$ and X have the same meanings as above;

then coupling with an amino-acid, an aminoester, an amino-amide or a peptide, if necessary protected,

and if necessary eliminating the protecting groups,

the reaction of the monoester of formula (I) with the compound of formula (II) being carried out without any mineral base when $R_1$ is an arylmethyl group.

2. The method of claim 1, characterized in that $R_1$ represents an allyl radical of formula (V):

```
R7
  \
   C=C-CH2-                  (V)
  /  |
R8   R9
```

wherein $R_7$, $R_8$ and $R_9$, which are the same or different, represent a hydrogen atom, a halogen atom, a branched or linear alkyl radical having from 1 to 6 carbon atoms, or a phenyl radical.

3. The method of any of claims 1 or 2, characterized in that the aspartic acid monoester of formula (I) is caused to react with the dicarbonyl compound of formula (II) in the presence of a secondary or tertiary amine.

4. The method of claim 3, characterized in that the secondary or tertiary amine is selected from diisopropylamine, N-methylcyclohexylamine, dicyclohexylamine, N-2,2,6,6-tetramethylpiperidine, N-methyl D- or L-α-methylbenzylamine, triethylamine, tributylamine, N-methylmorpholine, N-methylpiperidine and N,N-dimethyl D- or L-α-methylbenzylamine.

5. The method of any of claims 1 to 4, characterized in that the reaction of the aspartic acid monoester of formula (I) with the β-dicarbonyl compound of formula (II) is carried out in a solvent selected from an alcohol, a cyclic or linear ether, an ester, an aromatic or nonaromatic hydrocarbon, a ketone, a halogen derivative or a polar aprotic solvent such as dimethylformamide, dimethylacetamide, N-methylpyrrolidone, dimethylsulfoxide, hexamethylphosphotriamide or any mixture of said solvents.

6. The method of any of claims 1 or 2, characterized in that the aspartic acid monoester of formula (I) is selected from β-L-aspartic benzyl ester, β-L-aspartic methyl ester, β-L-aspartic tert-butyl ester and β-L-aspartic allyl ester.

7. The method of claim 1, characterized in that the dicarbonyl compound of formula (II) is methyl

acetoacetate, ethyl acetoacetate, isopropyl acetoacetate or butyl acetoacetate.

8. The method of claim 2, characterized in that the protecting group at the β-position of the aspartic residue is eliminated by protolysis in the presence of a transition metal catalyst.

9. The method of claim 8, characterized in that the deprotection is carried out in a protic medium at a temperature between -30° C and +100° C.

10. The method of any of claims 8 or 9, characterized in that the transition metal is palladium or ruthenium.

11. New aspartic acid derivatives useful in the synthesis of aspartyl peptides, comprising the enamines of formula (III):

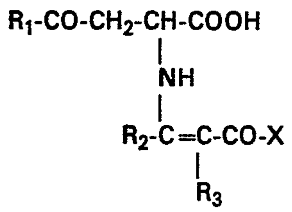

(III)

wherein $R_1$ represents a hydrocarbon radical possibly substituted by an activating group, $R_2$ represents a hydrocarbon radical, a carbomethoxymethyl radical or a carboethoxyethyl radical; $R_3$ is a hydrogen atom or $R_2$ and $R_3$ are combined to form a $-(CH_2)_n-$ chain wherein n is 3 or 4; X represents a hydrocarbon radical or X and $R_2$ are combined to form a three-carbon chain possibly substituted (and in such case, $R_3$ is a hydrogen atom), or X is an alkoxy radical $-OR_4$, where $R_4$ is a hydrocarbon radical or is a $-NR_5R_6$ radical where $R_5$ and $R_6$, which are the same or different, represent a hydrogen atom, a hydrocarbon radical possibly substituted, or are combined to form a 5 or 6-membered ring which may comprise an oxygen atom, and the amine salts thereof.

12. The aspartic acid derivatives of claim 11, characterized in that $R_1$ represents an allyl radical of formula (V):

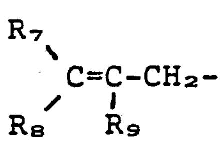

(V)

wherein $R_7$, $R_8$ and $R_9$, which are the same or different, represent a hydrogen atom, a halogen atom, a branched or linear alkyl radical having from 1 to 6 carbon atoms, or a phenyl radical.

13. The aspartic acid derivatives of claim 12, characterized in that $R_7$, $R_8$, and $R_9$ represent a hydrogen atom.

14. The derivatives of claim 11, characterized in that they are in the form of secondary or tertiary amine salts.